# EUROPEAN PATENT APPLICATION

(11) **EP 2 092 887 A1**
(43) Date of publication of application: **26.08.2009**
(21) Application number: 07829347.9
(22) Date of filing: 05.10.2007
(51) Int. Cl.: A61B 5/151

(54) **PUNCTURE INSTRUMENT**

(30) Priority: 13.12.2006 JP 2006336303
(71) Applicant: Terumo Kabushiki Kaisha, Shibuya-ku Tokyo 151-0072 (JP)
(72) Inventor: TAKINAMI, Masao, Nakakoma-gun Yamanashi 409-3853 (JP); TAKEMOTO, Masafumi, Nakakoma-gun Yamanashi 409-3853 (JP)
(74) Representative: TBK-Patent
(86) International application number: PCT/JP2007/069609
(87) International publication number: WO 2008/072414

(57) **Abstract**

A puncture instrument that before use, is detachably fitted to a fitting area of puncture device. The puncture instrument is one comprising a puncture needle having a needle member provided at its distal end with a sharp needle tip and a needle hub fixed to the proximal end of the needle member; a casing adapted to accommodate the puncture needle in a fashion allowing movement thereof in its longitudinal direction; and an extension part movably fitted to the casing, the extension part adapted to move relative to the casing so as to allow extension of the length of the casing on its proximal end side, wherein in the state of nonextension of the extension part, regular fitting to the puncture device can be realized, and wherein in the state of extension of the extension part, regular fitting to the puncture device cannot be realized.

## Description

### Technical Field

The present invention relates to a puncture instrument.

### Background Art

Attendant on the increase in the number of diabetics, in recent years, self-monitoring of blood glucose in which daily variations in a patient's blood glucose level are monitored by the patient himself or herself has been recommended.

For the blood glucose measurement, a blood glucose measuring instrument for automatically measuring the quantity of glucose in blood is used. Prior to the measurement, the patient must take a sample of his or her own blood.

The blood sample is taken by a method in which a fingertip skin is punctured with a puncture needle, and then the periphery of the punctured portion is depressed by a finger or the like to squeeze out the blood.

The puncture of the fingertip with the puncture needle is conducted by use of a puncture device including a puncture needle cartridge, and a puncture device body for receiving the puncture needle cartridge detachably attached to a distal portion thereof (see, for example, Patent Document 1). Specifically, the puncture needle cartridge is attached to the puncture device body, and the puncture device body is actuated, to thereby cause the puncture needle of the puncture needle cartridge to protrude, puncturing the fingertip. Besides, after the puncture of the fingertip, i.e. after use, the puncture needle cartridge is removed from the puncture device body.

This puncture device has such a configuration that the puncture needle cartridge can be attached to the puncture device body, irrespectively of whether the puncture needle cartridge is an unused one or a used one. Therefore, there has been a fear that a used puncture needle cartridge might be attached to the puncture device body, by mistake, and a fingertip might be punctured with the used puncture needle cartridge.

Patent Document 1: Japanese Patent Laid-Open No. 2000-245717

### Disclosure of Invention

It is an object of the present invention to provide a puncture instrument with which it is possible to securely prevent a used puncture instrument from being attached to a puncture device and used again.

In order to attain the above object, according to the present invention, there is provided
a puncture instrument to be used in the state of being detachably attached to an attaching part of a puncture device, including:
a puncture needle which has a needle member provided with a sharp needle tip at its distal end, and a needle hub fixed to a proximal portion of the needle member;
a casing adapted to store the puncture needle movably in the longitudinal direction thereof; and
an extension part movably disposed on the casing and moved relative to the casing so as to allow the length of the casing on the proximal side to extend;
wherein in a non-extension condition where the extension part is not extended, normal attachment to the puncture device is possible, and in an extension condition where the extension part is extended, normal attachment to the puncture device is impossible.

This makes it possible to securely prevent a used puncture instrument from being again attached to the puncture device and used.

In addition, in the puncture instrument according to the present invention, preferably,
the puncture device has a plunger adapted to be connected to the needle hub when the puncture instrument is attached to the attaching part; and
in the extension condition, the increase in the length of the extension part prevents the needle hub from reaching the plunger, and the needle hub and the plunger cannot be connected to each other.

This ensures that normal attachment of a used puncture instrument to the puncture device cannot be realized, so that a used puncture instrument can be securely prevented from being reused.

Besides, in the puncture instrument according to the present invention, preferably, the extension part is brought into the extension condition when the puncture instrument, once attached to the attaching part in the non-extension condition, is detached from the attaching part.

This results in that an unused puncture instrument and a used puncture instrument are different from each other in overall length. This makes it impossible for a used puncture instrument to be attached to the puncture device. Besides, whether a puncture instrument is an unused one or a used one can be assuredly discriminated visually, so that a used puncture instrument can be securely prevented from being reused by mistake.

In addition, the puncture instrument according to the present invention, preferably, includes non-extension condition maintaining means for maintaining the non-extension condition.

By this, unwilling movement of the extension part, specifically, unwilling displacement of the extension part from the non-extension condition into the extension condition, is prevented from occurring.

Besides, in the puncture instrument according to the present invention, preferably, the non-extension condition maintaining means includes a projected portion provided in one of the casing and the extension part, and a recessed portion which is provided in the other of the casing and the extension part and with which the projected portion is adapted to be engaged.

By this, unwilling movement of the extension part, specifically, unwilling displacement of the extension part from the non-extension condition into the extension condition, is prevented from occurring.

In addition, the puncture instrument according to the present invention, preferably, includes extension condition maintaining means for maintaining the extension condition.

This makes it possible to securely maintain the extension condition, specifically, to prevent the extension part in the extension condition from unwillingly entering into the casing to get into the non-extension condition.

Besides, in the puncture instrument according to the present invention, preferably, the extension condition maintaining means includes a projected portion provided in one of the casing and the extension part, and a recessed portion which is provided in the other of the casing and the extension part and with which the projected portion is adapted to be engaged.

This makes it possible to securely maintain the extension condition, specifically, to prevent the extension part in the extension condition from unwillingly entering into the casing to get into the non-extension condition.

In addition, in the puncture instrument according to the present invention, preferably, the extension part has a first engaging portion adapted to be engaged with the attaching part when the puncture instrument is attached to the attaching part, and a second engaging portion adapted to be engaged with the casing in the non-extension condition; and
an engaging force between the first engaging portion and the attaching part is greater than an engaging force between the second engaging portion and the casing.

This ensures that when the puncture instrument (casing) attached to the puncture device is pulled in the distal direction, first, the casing is moved in the distal direction precedential to the extension part; specifically, the casing is moved in the distal direction but the extension part is left indwelling in the attaching part. When the puncture instrument (casing) is pulled further in the distal direction, the movement of the casing relative to the extension part reaches a limit, and the extension condition results.

Besides, in the puncture instrument according to the present invention, preferably, the extension part and the casing are tubular in shape.

This ensures that when the extension part is moved relative to the casing, the movement is performed assuredly.

In addition, in the puncture instrument according to the present invention, preferably, the extension part is disposed so as to be slidable on an inner peripheral part of the casing.

This ensures that when the extension part is moved relative to the casing, the movement is performed assuredly.

Besides, in the puncture instrument according to the present invention, preferably, the extension part is disposed so as to be slidable on an outer peripheral part of the casing.

This ensures that when the extension part is moved relative to the casing, the movement is performed assuredly.

In addition, in the puncture instrument according to the present invention, preferably, the casing and the extension part have different colors.

This ensures that in the case of the extension condition, the extension part can be visually confirmed more assuredly than in the non-extension condition. This makes it possible to reliably recognize that the puncture instrument is a used one, and, therefore, to prevent more reliably the used puncture instrument from being reused by mistake.

Besides, in the puncture instrument according to the present invention, preferably, the casing has a colored part having a portion which is covered with the extension part in the non-extension condition and is exposed in the extension condition, the portion being colored.

This ensures that in the case of the extension condition, the colored part is exposed, so that the colored part can be visually confirmed. This makes it possible to reliably recognize that the puncture instrument is a used one, and, therefore, to prevent more reliably the used puncture instrument from being reused by mistake.

### Brief Description of Drawings

[FIG. 1]

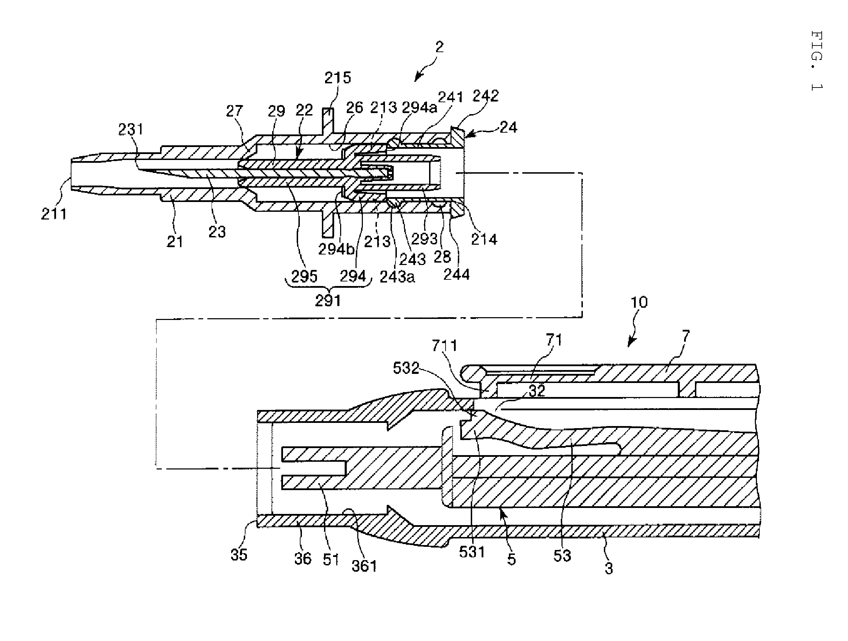

   FIG. 1 is a longitudinal sectional view showing a first embodiment of the puncture instrument according to the present invention (a view showing the condition before attachment of the puncture instrument to a puncture device).
[FIG. 2]

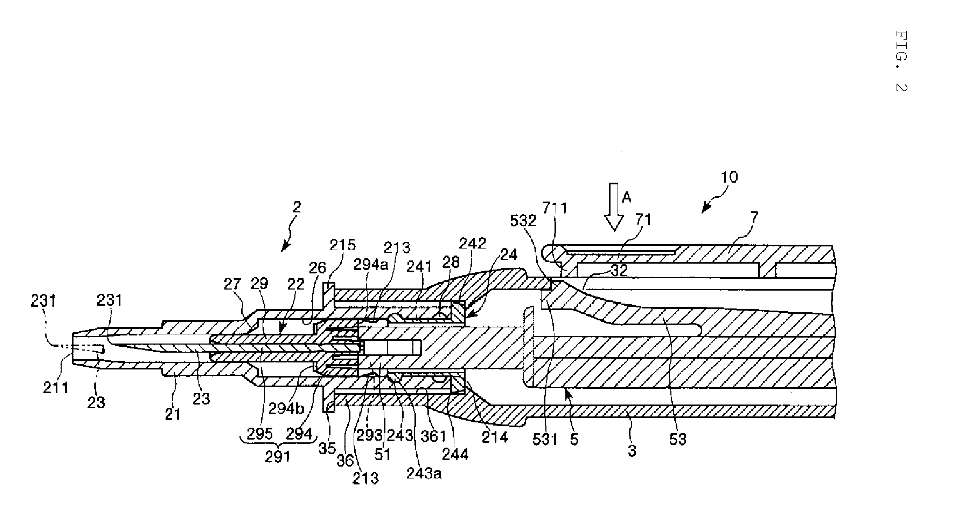

   FIG. 2 is a longitudinal sectional view showing the first embodiment of the puncture instrument according to the present invention (a view showing the condition where the puncture instrument is normally attached to the puncture device).
[FIG. 3]

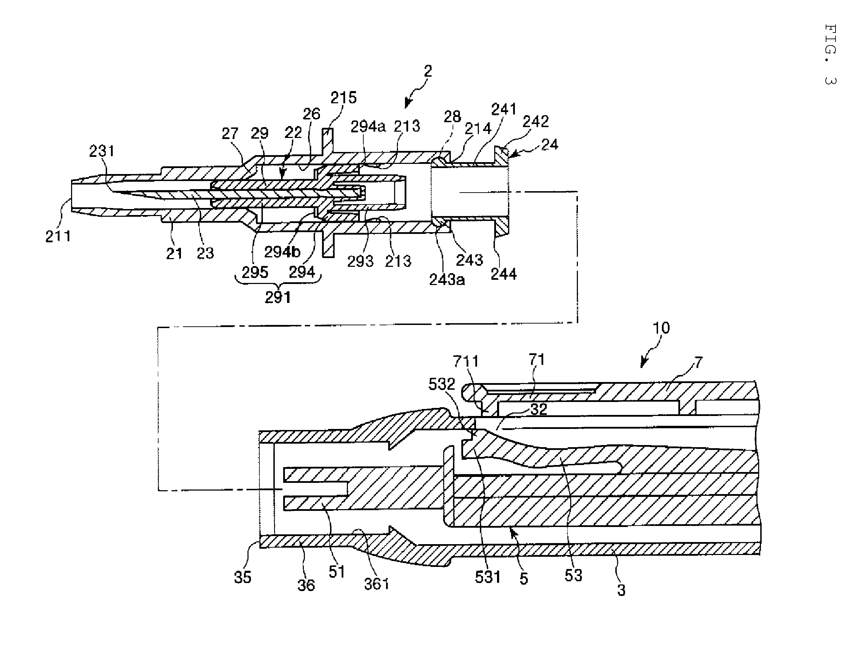

   FIG. 3 is a longitudinal sectional view showing the first embodiment of the puncture instrument according to the present invention (a view showing the condition where the puncture instrument is detached from the puncture device).
[FIG. 4]

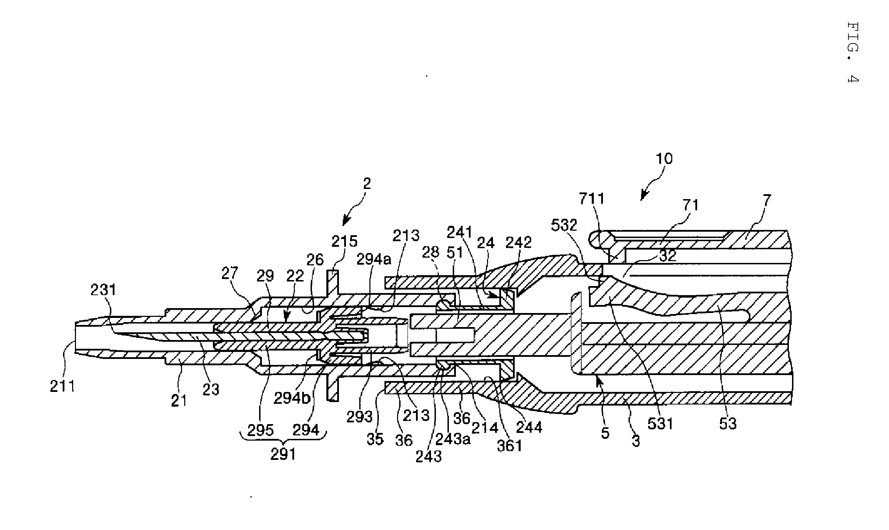

   FIG. 4 is a longitudinal sectional view showing the first embodiment of the puncture instrument according to the present invention (a view showing the condition where the puncture instrument has failed to be normally attached to the puncture device).
[FIG. 5]

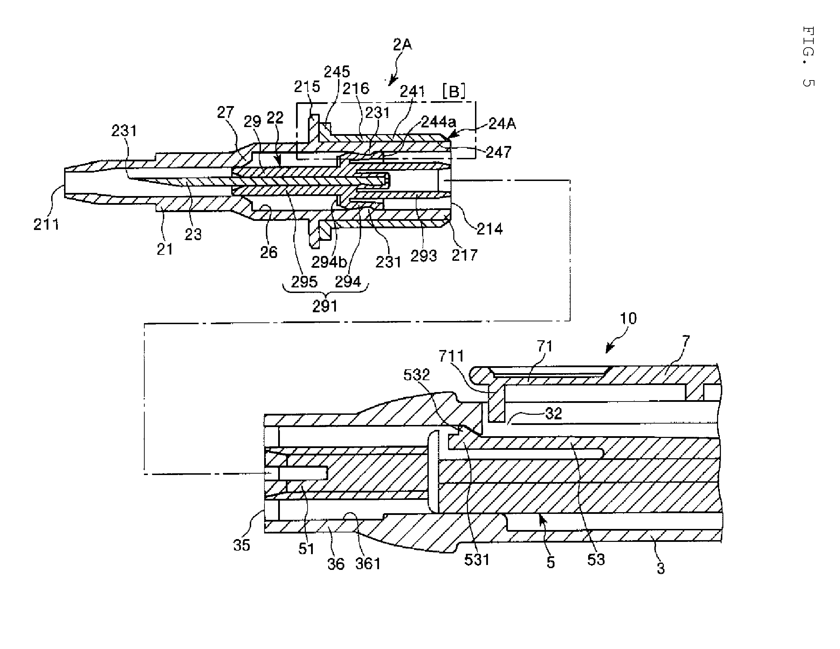

   FIG. 5 is a longitudinal sectional view showing a second embodiment of the puncture instrument according to the present invention (a view showing the condition before attachment of the puncture instrument to a puncture device).
[FIG. 6]

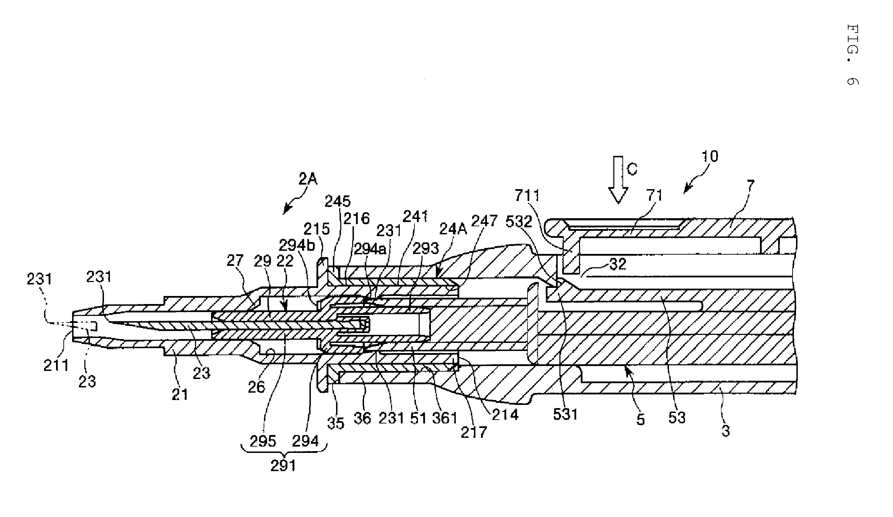

   FIG. 6 is a longitudinal sectional view showing the second embodiment of the puncture instrument according to the present invention (a view showing the condition where the puncture instrument is normally attached to the puncture device).
[FIG. 7]

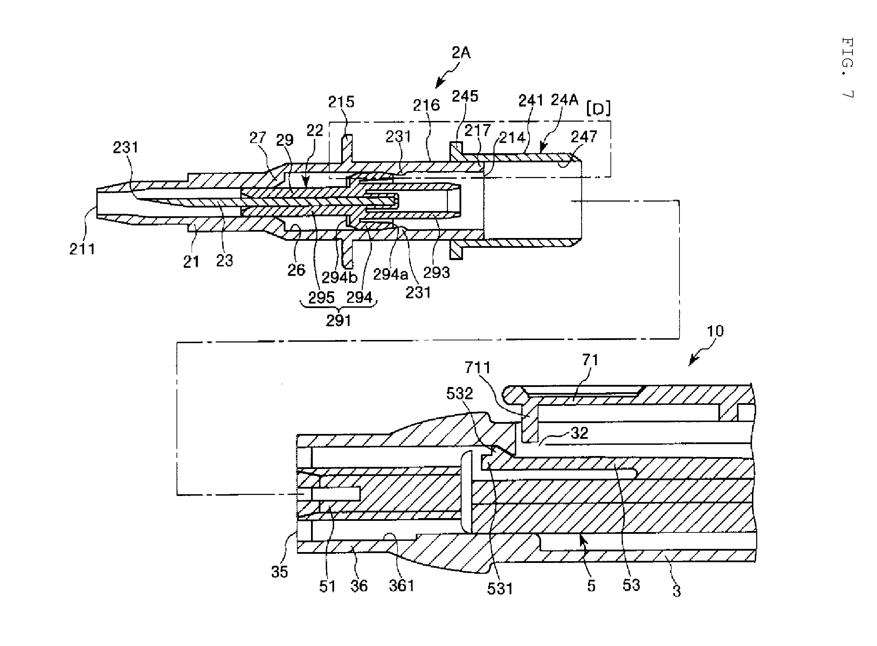

   FIG. 7 is a longitudinal sectional view showing the second embodiment of the puncture instrument according to the present invention (a view showing the condition where the puncture instrument is detached from the puncture device).
[FIG. 8]

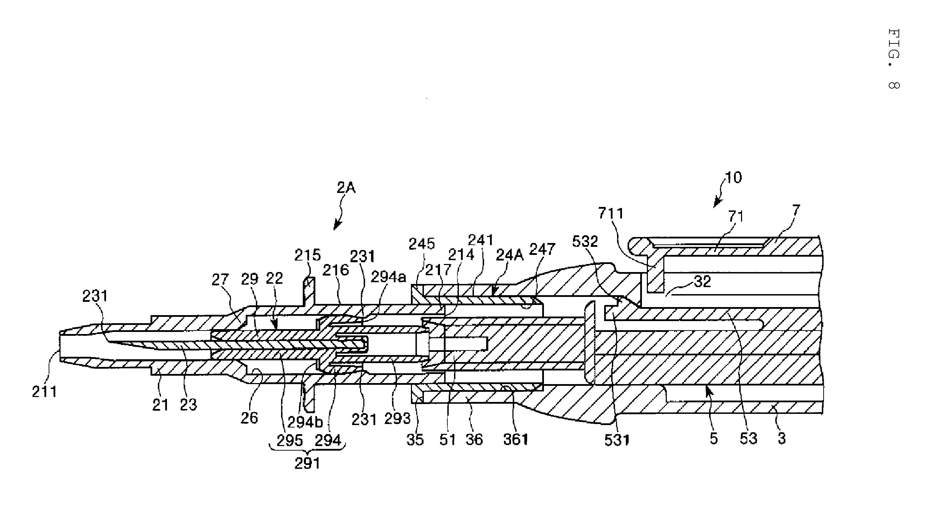

   FIG. 8 is a longitudinal sectional view showing the second embodiment of the puncture instrument according to the present invention (a view showing the condition where the puncture instrument has failed to be normally attached to the puncture device).
[FIG. 9]

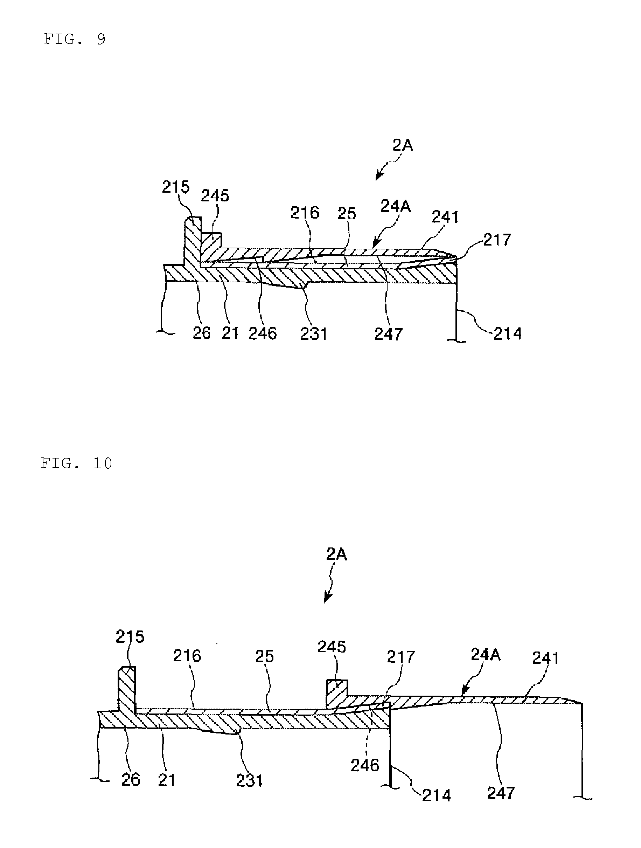

   FIG. 9 is an enlarged detailed view of region [B] surrounded by dot-dash line in FIG. 5.
[FIG. 10]
   FIG. 10 is an enlarged detailed view of region [D] surrounded by dot-dash line in FIG. 7.

### Best Mode for Carrying Out the Invention

Now, the puncture instrument according to the present invention will be described in detail below, based on preferred embodiments shown in the attached drawings.

### <First Embodiment>

FIGS. 1 to 4 are longitudinal sectional views showing a first embodiment of the puncture instrument according to the present invention (FIG. 1 is a view showing the condition before attachment of the puncture instrument to a puncture device, FIG. 2 is a view showing the condition where the puncture instrument is normally attached to the puncture device, FIG. 3 is a view showing the condition where the puncture instrument is detached from the puncture device, and FIG. 4 is a view showing the condition where the puncture instrument has failed to be normally attached to the puncture device).
Incidentally, for convenience of description, the right side in FIGS. 1 to 4 (and also in FIGS. 5 to 10) will hereinafter be referred to as "proximal (end)," and the left side as "distal (end)."

The puncture instrument 2 shown in FIGS. 1 to 4 is used in the state of being detachably attached to a puncture device 10.

Before description of the puncture instrument 2, the puncture device 10 will be described.
The puncture device 10 has a housing 3, a plunger 5, and a puncture operating part (operating means) 7.

The housing 3 includes a long hollow cylindrical body. To a distal portion (attaching part) 36 of the housing 3, a casing 21 of the puncture instrument 2 is detachably attached.

In addition, on the upper side in FIG. 1 (and also in FIGS. 2 to 4) of the housing 3, the puncture operating part 7 is provided, which is elastically deformable and is plate-like in shape. At a distal portion of the puncture operating part 7, there is formed an operating button 71 which has a projection 711 projecting to the lower side in FIG. 1. In addition, the housing 3 is provided, at a position corresponding to the projection 711, with an opening 32 which is larger than the projection 711 and smaller than the operating button 71.

The plunger 5 is stored in the housing 3. The plunger 5 is a part of which a distal portion 51 is connected to a hub 29 (attached part 293) of a puncture needle 22 of the puncture instrument 2 when the puncture instrument 2 is attached to the housing 3 (see FIG. 2).

In addition, the plunger 5 is movable along its longitudinal direction in the housing 3, by a puncturing coil spring (not shown) for urging the plunger 5 in the distal direction and a returning coil spring (not shown) for urging (returning) the plunger 5 in the proximal direction. By the movements of the plunger 5, the puncture needle 22 of the puncture instrument 2 can assuredly be moved, specifically, puncturing with the puncture needle 2 can be performed.

Besides, on the upper side in FIG. 1 of the plunger 5, there is provided a rod-like lock member 53 which is elastically deformable. The lock member 53 is provided at its distal portion with a lock part 531 which has a projection 532 projecting to the upper side in FIG. 1.

When the puncture instrument 2 is mounted in position, i.e., in the condition where preparation (setting) for puncture is completed, the projection 532 of the lock part 531 is inserted in the opening 32 in the housing 3, with the plunger 5 urged in the distal direction relative to the housing 3 by the puncturing coil spring, whereby the lock part 531 is locked relative to the housing 3 (see FIG. 2).

When the operating button 71 of the puncture operating part 7 is pressed in the direction of arrow A in FIG. 2 under the condition shown in FIG. 2, the projection 711 presses against the projection 532 of the lock member 53, whereby locking of the lock part 531 relative to the housing 3 is released. This results in that the plunger 5 is moved in the distal direction by an urging force of the puncturing coil spring.

Now, the puncture instrument 2 will be described below.
The puncture instrument 2 shown in FIG. 1 (and also in FIGS. 2 to 4) includes a tubular casing (puncture needle holder) 21, a puncture needle 22 stored in the casing 21 so as to be movable in the axial direction (longitudinal direction), and an extension part 24 movably disposed in the casing 21. Now, configurations of the components will be described below.

As shown in FIG. 1, the puncture needle 22 includes a needle member 23, and a needle hub (hereinafter referred to simply as "hub") 29.

The needle member 23 is provided (formed) with a sharp needle tip 231 at its distal end.
The material constituting the needle member 23 is not particularly limited. Examples of the material include various metallic materials such as stainless steel, aluminum, aluminum alloys, titanium, titanium alloys, and superelastic alloys, e.g., Ni-Ti alloy, and various hard resin materials such as polyphenylene sulfide.

The hub 29 includes a columnar hub body 291, and an attached part 293 provided on the proximal side of the hub body 291 and attached (connected) to the plunger 5 of the puncture device 10.

The hub body 291 is fixed to a proximal portion of the needle member 23 so that the needle tip 231 of the needle member 23 is exposed. The hub body 291 includes a large diameter part 294, and a small diameter part 295 formed on the distal side of the large diameter part 294.

The large diameter part 294 has an outside diameter approximately equal to a maximum inside diameter of an inner peripheral part 26 of the casing 21.

The small diameter part 295 has an outside diameter smaller than the outside diameter of the large diameter part 294. In addition, the distal side (the needle tip 231) of the needle member 23 is exposed from a distal portion of the small diameter part 295.

The attached part 293 is hollow cylindrical in shape, and is formed to protrude to the proximal side beyond a proximal end face 294a of the large diameter part 294 of the hub body 291. Besides, the attached part 293 is formed to be smaller in outside diameter than the large diameter part 294.

Incidentally, the material constituting the hub 29 is not particularly limited. Examples of the material include polyolefins such as polyethylene, polypropylene, ethylene-vinyl acetate copolymer, etc., modified polyolefins, polyamides (e.g., 6-nylon, 46-nylon, 66-nylon, 610-nylon, 612-nylon, 11-nylon, 12-nylon, 6,12-nylon, 6,66-nylon), thermoplastic polyimides, liquid crystal polymers such as aromatic polyesters, etc., polyphenylene oxide, polyphenylene sulfide, polycarbonate, polymethyl methacrylate, polyethers, polyether ether ketones, polyether imides, polyacetal, and various thermoplastic elastomers based on styrene, polyolefin, polyvinyl chloride, polyurethane, polyester, polyamide, polybutadiene, trans-polyisoprene, fluoro-rubber, or chlorinated polyethylene, as well as copolymers, polymer blends, polymer alloys and the like based on these materials, which may be used either singly or in combination of two or more of them.

The casing 21 stores the puncture needle 22 therein. The distal end of the casing 21 is a part to be brought into contact with a living body surface such as, a fingertip, a palm, an upper arm, an abdominal part, a thigh or an earlobe, and is formed with an opening (tip opening) 211 through which the needle tip 231 of the puncture needle 22 can protrude. In addition, an opening (proximal opening) 214 is provided at the proximal end of the casing 21.

The casing 21 is provided at its inner peripheral part 26 with a stepped part 27 and a hub engaging part 213.

The stepped part 27 is so formed that the inside diameter of the inner peripheral part 26 is abruptly reduced there from the proximal side to the distal side. A distal end face 294b of the large diameter part 294 of the puncture needle 22 abuts on the stepped part 27, whereby the maximum protrusion amount of the puncture needle 22 (the needle tip 231) from the opening 211 in the casing 21 is limited.

The hub engaging part 213 is a part engageable with the outer periphery of the large diameter part 294 (the hub 29). In addition, the hub engaging part 213 is a part where a part of the inner peripheral part 26 is raised, and it is arranged as a rib (projected streak) along the circumferential direction of the inner peripheral part 26 of the casing 21. As shown in FIG. 1, the large diameter part 294 is fitted in (locked by) the hub engaging part 213, whereby unintended movement of the puncture needle 22, such as unintended protrusion of the needle tip 231 via the opening 211 of the casing 21, can be prevented from occurring.

In addition, the casing 21 is provided at its outer peripheral part with an annular rib (flange part) 215 which abuts on the distal end 35 of the housing 3 when the puncture instrument 2 is attached to the puncture device 10 (see, for example, FIG. 2).

Besides, the material constituting the casing 21 is not particularly limited; for example, the constituent materials mentioned in the description of the hub 29 above can be used as the material for the casing 21.

As shown in FIGS. 1 to 4, the extension part 24 is disposed at a proximal portion of the inner peripheral part 26 of the casing 21. The extension part 24 can be moved into a non-extension condition (see FIGS. 1 and 2) and an extension condition (see FIGS. 3 and 4). Herein, "the non-extension condition" means a condition where the extension part 24 is stored inside the inner peripheral part 26 of the casing 21, that is, a condition where the extension part 24 is not extended. On the other hand, "the extension condition" means a condition where the extension part 24 has moved in the proximal direction relative to the casing 21 so that the length of the casing 21 is extended in the proximal direction in appearance.

The extension part 24 is hollow cylindrical in shape, and is moved while sliding in the casing 21 (the inner peripheral part 26) at the time of displacement from the non-extension condition into the extension condition. In this instance, a sliding resistance is generated between a projected portion 243, projectingly provided at the outer peripheral part 241 of the extension part 24, and the inner peripheral part 26 of the casing 21. In addition, for example in the puncture instrument 2 in an unused state (the state shown in FIG. 1), the projected portion 243 of the extension part 24 is engaged with the inner peripheral part 26 of the casing 21. Therefore, unintended movement of the extension part 24, specifically, unintended displacement of the extension part 24 from the non-extension condition into the extension condition, is prevented from occurring.

Thus, in the puncture instrument 2, the outer peripheral part 241 (particularly, the projected portion 243) of the extension part 24 functions as an engaging part (second engaging part) which is engaged with the casing 21 in the non-extension condition. Therefore, it can be said that the outer peripheral part 241 of the extension part 24 and the inner peripheral part 26 of the casing 21 function as non-extension condition maintaining means for maintaining the non-extension condition.

Besides, the extension part 24 has a flange part 242 provided at its proximal portion. The flange part 242 is projectingly formed at the outer peripheral part 241 of the extension part 24.

The flange part 242 is a part having a maximum outside diameter set to be slightly larger than the outside diameter of the inner peripheral part 26 of the casing 21.

In addition, the flange part 242 has an outer peripheral surface, the outside diameter of which is gradually decreased along the proximal direction. As a result, an angular part 244 (first engaging part) forming an acute angle is formed at the distal end of the flange part 242. As shown in FIG. 2, when the puncture instrument 2 is attached to the puncture device 10 (the distal portion 36), the angular part 244 is engaged with an inner peripheral surface 361 of the distal portion 36 of the puncture device 10.

The projected portion 243 is a part having an outside diameter set to be slightly larger than the inside diameter of the inner peripheral part 26 of the casing 21. Besides, the projected portion 243 has an outer peripheral surface 243a which is rounded in shape.

The inner peripheral part 26 of the casing 21 is provided with a recessed portion 28 at a position corresponding to the projected portion 243 of the extension part 24 in the extension condition (see FIGS. 3 and 4). In the extension condition, the projected portion 243 of the extension part 24 is in engagement with the recessed portion 28 of the casing 21. This makes it possible to securely maintain the extension condition, specifically, to prevent the extension part 24 in the extension condition from accidentally entering into the casing 21 with the result of the non-extension condition. Thus, it can be said that the projected portion 243 of the extension part 24 and the recessed portion 28 of the casing 21 function as extension condition maintaining means for maintaining the extension condition.

In the puncture instrument 2 configured as above, the puncture instrument 2 in an unused state, i.e., before attached to the puncture device 10, is in the non-extension condition (see FIG. 1). Besides, the puncture instrument 2 in a used state, i.e., after detached from the puncture device 10, is in the extension condition (see FIG. 3).

When the puncture instrument 2, starting from the state shown in FIG. 1, is inserted into the distal portion 36 of the puncture device 10 until the rib 215 of the casing 21 thereof abuts on the distal end 35 of the puncture device 10 (the housing 3), the distal portion 51 of the plunger 5 is passed through the extension part 24, and the attached part 293 of the puncture needle 22 is fitted into the distal part 51 (see FIG. 2).

Besides, in this instance, the attached part 293 of the puncture needle 22 pushes the distal portion 51 of the plunger 5 in the proximal direction, against the urging force of the puncturing coil spring. Attendant on the proximal movement of the plunger 5, the lock member 53 is also moved in the proximal direction. As a result, the projection 532 of the lock member 53 is put in the opening 32 of the housing 3, and the lock part 531 is locked relative to the housing 3 (see FIG. 2).

In addition, when the puncture instrument 2 is further pushed in, the puncture needle 22 of the puncture instrument 2 is pushed in the distal direction by a reaction force exerted from the plunger 5, whereby engagement between the large diameter part 294 of the puncture needle 22 and the hub engaging part 213 of the casing 21 is released (see FIG. 2).

Thus, in the non-extension condition, the puncture instrument 2 can be attached to the puncture device 10; in other words, preparation (setting) for puncture can be performed.

When the operating button 71 is pushed in the direction of arrow A in FIG. 2 under the condition shown in FIG. 2, the locking of the lock part 531 to the housing 3 is released, as above-mentioned, and the plunger 5 is moved in the distal direction by the urging force of the puncturing coil spring. As a result, the puncture needle 22 is moved in the distal direction, and the needle tip 231 is made to protrude from the opening 211 of the casing 21 (see the part indicated by two-dotted chain lines in FIG. 2). Thus, a living body surface can be punctured with the needle tip 231 made to protrude.

Besides, thereafter, the plunger 5 is urged in the proximal direction by the returning coil spring, to be returned into the state shown in FIG. 1. This results in that the puncture needle 22 is also moved in the proximal direction, so that the puncture needle 22 is wholly stored in the casing 21.

Starting from the condition shown in FIG. 2 after the puncture, the puncture instrument 2 is pulled in the distal direction, to be detached from the puncture device 10 (the distal portion 36) (see FIG. 3). In this instance, the extension part 24 is moved in the proximal direction, resulting in the extension condition.

For obtaining the extension condition, the engaging force between the angular part 244 of the extension part 24 and the inner peripheral surface 361 of the distal portion 36 of the housing 3 is preferably set to be larger than the sliding resistance (engaging force) between the outer peripheral part 241 of the extension part 24 and the inner peripheral part 26 of the casing 21. This ensures that when the puncture instrument 2 (the casing 21) is pulled in the distal direction, first, the casing 21 is moved in the distal direction precedential to the extension part 24, specifically, the casing 21 is moved in the distal direction but the extension part 24 is left indwelling in situ (in the position shown in FIG. 2). When the puncture instrument 2 (the casing 21) is pulled further in the distal direction, the projected portion 243 of the extension part 24 and the recessed portion 28 of the casing 21 are engaged with each other.

Through such a process, the puncture instrument 2 is securely brought into the extension condition. In addition, as above-mentioned, the extension condition is securely maintained by the engagement between the projected portion 243 of the extension part 24 and the recessed portion 28 of the casing 21.

Incidentally, the method by which the engaging force between the angular part 244 of the extension part 24 and the inner peripheral surface 361 of the distal portion 36 of the housing 3 is set to be larger than the sliding resistance between the outer peripheral part 241 of the extension part 24 and the inner peripheral part 26 of the casing 21 is not particularly limited. Examples of the method include a method in which the fit tolerance between the angular part 244 of the extension part 24 and the inner peripheral surface 361 of the distal portion 36 of the housing 3 and the fit tolerance between the outer peripheral part 241 of the extension part 24 and the inner peripheral part 26 of the casing 21 are respectively set appropriately. By this, the magnitude relationship between the two forces can be set assuredly.

Here, it is assumed that it is attempted to attach the puncture instrument 2 in the extension state, i.e. in the used state, again to the puncture device 10, as shown in FIG. 4. In this case, the length of the puncture instrument 2 on the proximal side has been enlarged by an amount equal to the increase in the length of the extension part 24, and, therefore, the attached part 293 of the hub 29 does not reach the distal portion 51 of the plunger 5. Consequently, connection of the attached part 293 of the hub 29 to the distal portion 51 of the plunger 5, that is, normal attachment of the puncture instrument 2 to the puncture device 10, is impossible.

Thus, in the case where the puncture instrument 2 is a used one, the puncture instrument 2 is securely prevented from being again attached to the puncture device 10, so that reuse of the used puncture instrument 2 is prevented assuredly.

In addition, an unused puncture instrument 2 and a used puncture instrument 2 are different from each other in the position of the extension part 24 relative to the casing 21 and, therefore, in overall length (see FIGS. 1 and 3). This ensures that the state of the puncture instrument 2, specifically, whether the puncture instrument 2 is an unused one or a used one, can be discriminated visually and reliably. Accordingly, the used puncture instrument 2 can be securely prevented from being reused by mistake.

Besides, the extension part 24 and the casing 21, preferably, have different colors. This ensures that in the case of the extension condition, the extension part 24 can be visually confirmed more assuredly than in the non-extension condition. Thus, the puncture instrument 2 can securely be recognized to be used. Therefore, the used puncture instrument 2 can be more reliably prevented from being used again by mistake.

Incidentally, the material constituting the extension part 24 is not particularly limited. For example, the constituent materials mentioned in the description of the hub 29 above can be used as the material for the extension part 24.

### <Second Embodiment>

FIGS. 5 to 8 are longitudinal sectional views showing a second embodiment of the puncture instrument according to the present invention (FIG. 5 is a view showing the condition before attachment of the puncture instrument to a puncture device, FIG. 6 is a view showing the condition where the puncture instrument is normally attached to the puncture device, FIG. 7 is a view showing the condition where the puncture instrument is detached from the puncture device, and FIG. 8 is a view showing the condition where the puncture instrument has failed to be attached to the puncture device), FIG. 9 is an enlarged detailed view of region [B] surrounded by dot-dash line in FIG. 5, and FIG. 10 is an enlarged detailed view of region [D] surrounded by dot-dash line in FIG. 7. Incidentally, in each of FIGS. 9 and 10, the puncture needle is omitted.

Now, the second embodiment of the puncture instrument according to the present invention will be described referring to these figures. The following description will be centered on the differences of this embodiment from the above-described embodiment, and descriptions of the same items as above will be omitted.

This embodiment is the same as the first embodiment above, except for a difference in the layout position of the extension part.
In the puncture instrument 2A shown in FIGS. 5 to 8, an extension part 24A is disposed at an outer peripheral part 216 of a casing 21. The extension part 24A is hollow cylindrical in shape, and its inner peripheral part 247 (second engaging part) slides on an outer peripheral part 216 of the casing 21.

The extension part 24A has a flange part 245 and a recessed portion 246 provided at its distal portion (see FIGS. 9 and 10) .

The flange part 245 is projectingly formed at an outer peripheral part 241 (first engaging part) of the extension part 24A. The flange part 245 is a part where the outside diameter of the outer peripheral part 241 is enlarged. In addition, as shown in FIG. 6, the flange part 245 abuts on the distal end 35 of the puncture device 10 when the puncture instrument 2A is attached to the puncture device 10.

The recessed portion 246 is formed in an inner peripheral part 247 of the extension part 24A. The recessed portion 246 is a portion which is wedge-like in longitudinal sectional shape, specifically, a part which is gradually increased in depth (is tapered) along the proximal direction.

A proximal portion 217 of the casing 21 is gradually increased in outside diameter (is tapered) along the proximal direction. In the extension condition, the recessed portion 246 of the extension part 24A is engaged with the proximal portion 217 (projected portion) of the casing 21 (see FIG. 10). By this, the extension condition can be maintained securely. In other words, the extension part 24A can be prevented from unintendedly entering into the casing 21. Thus, it can be said that the recessed portion 246 of the extension part 24A and the proximal portion 217 of the casing 21 function as extension condition maintaining means for maintaining the extension condition.

When the puncture instrument 2A, starting from the state shown in FIG. 5, is inserted into a distal portion 36 of the puncture device 10 until the flange part 245 of the extension part 24A thereof abuts on the distal end 35 of the puncture device 10 (the housing 3), a distal portion 51 of a plunger 5 enters into the casing 21, and an attached part 293 of a puncture needle 22 is fitted into the distal portion 51 (see FIG. 6). Besides, attendant on this, the outer peripheral part 241 of the extension part 24A slides on an inner peripheral surface 361 of the distal portion 36.

In addition, when the puncture instrument 2A is inserted into the puncture device 10, the attached part 293 of the puncture needle 22 pushes the distal portion 51 of the plunger 5 in the proximal direction, against an urging force of the above-mentioned puncturing coil spring. Attendant on the proximal movement of the plunger 5, a lock member 53 is also moved in the proximal direction. This ensures that a projection 532 of the lock member 53 enters into an opening 32 of the housing 3, and a lock part 531 is locked relative to the housing 3 (see FIG. 6).

Besides, with the puncture instrument 2A further pushed in, the puncture needle 22 is pushed in the distal direction against a reaction force exerted from the plunger 5, whereby engagement between a large diameter part 294 of the puncture needle 22 and a hub engaging part 213 of the casing 21 is released (see FIG. 6).

Thus, in the non-extension condition, the puncture instrument 2A can be attached to the puncture device 10, in other words, preparation (setting) for puncture can be performed.

When an operating button 71 is pressed in the direction of arrow C in FIG. 6 under the condition shown in FIG. 6, locking of a lock part 531 to the housing 3 is released, and the plunger 5 is moved in the distal direction by the urging force of the above-mentioned puncturing coil spring. By this, the puncture needle 22 is moved in the distal direction, and a needle tip 231 is made to protrude from an opening 211 of the casing 21 (see the part indicated by two-dotted chain line in FIG. 6). With the needle tip 231 thus made to protrude, a living body surface can be punctured.

Besides, thereafter, the plunger 5 is urged in the proximal direction by the above-mentioned returning coil spring, to be returned into the state shown in FIG. 5. As a result of this, the puncture needle 22 is also moved in the proximal direction, so that the puncture needle 22 is wholly stored in the casing 21.

Starting from the condition shown in FIG. 6 after the puncture, the puncture instrument 2A is pulled in the distal direction, to be detached from the puncture device 10 (the distal portion 36) (see FIG. 7). In this case, the extension part 24A of the puncture instrument 2A is moved in the proximal direction, resulting in the extension condition.

For obtaining the extension condition, the sliding resistance (engaging force) between the outer peripheral part 241 of the extension part 24A and the inner peripheral surface 361 of the distal portion 36 of the housing 3 is preferably set to be larger than the sliding resistance (engaging force) between the inner peripheral part 247 of the extension part 24A and the outer peripheral part 216 of the casing 21. This ensures that when the puncture instrument 2A (the casing 21) is pulled in the distal direction, first, the casing 21 is moved in the distal direction precedential to the extension part 24A, specifically, the casing 21 is moved in the distal direction but the extension part 24A is left indwelling in situ (in the position shown in FIG. 6). When the puncture instrument 2A (the casing 21) is pulled further in the distal direction, the recessed portion 246 of the extension part 24A and the proximal portion 247 of the casing 21 are engaged with each other.

Through such a process, the puncture instrument 2A is securely brought into the extension condition. In addition, as above-mentioned, the extension condition is securely maintained by the engagement between the recessed portion 246 of the extension part 24A and the proximal portion 217 of the casing 21.

Incidentally, the method by which the sliding resistance between the outer peripheral part 241 of the extension part 24A and the inner peripheral surface 361 of the distal portion 36 of the housing 3 is set to be larger than the sliding resistance between the inner peripheral part 247 of the extension part 24A and the outer peripheral part 216 of the casing 21 is not particularly limited. Examples of the method include a method in which the fit tolerance between the outer peripheral part 241 of the extension part 24A and the inner peripheral surface 361 of the distal portion 36 of the housing 3 and the fit tolerance between the inner peripheral part 247 of the extension part 24A and the outer peripheral part 216 of the casing 21 are respectively set appropriately. By this, the magnitude relationship between the two forces can be set assuredly.

Here, it is assumed that it is attempted to attach the puncture instrument 2A in the extension state, i.e. in the used state, again to the puncture device 10, as shown in FIG. 8. In this case, the length of the puncture instrument 2A on the proximal side has been enlarged by an amount equal to the increase in the length of the extension part 24A, and, therefore, the attached part 293 of the hub 29 does not reach the distal portion 51 of the plunger 5 when the flange part 245 abuts on the distal end 35. Consequently, connection of the attached part 293 of the hub 29 to the distal portion 51 of the plunger 5, that is, normal attachment of the puncture instrument 2A to the puncture device 10, is impossible.

Thus, in the case where the puncture instrument 2A is a used one, the puncture instrument 2A is securely prevented from being again attached to the puncture device 10, so that reuse of a used puncture instrument 2A is prevented assuredly.

In addition, the outer peripheral part 216 of the casing 21 can be divided into the part which is covered with the extension part 24A in the non-extension condition and exposed in the extension condition, that is, the part on the proximal side of the rib 215, and the other part of the casing 21 exclusive of the just-mentioned part, that is, the part on the distal side of the rib 215. Of the puncture instrument 2A, preferably, the part on the proximal side of the rib 215 is colored in a color different from the color of the part on the distal side of the rib 215. Hereinafter, that part on the proximal side of the rib 215 which is thus colored will be referred to as "the colored part 25" (see FIG. 10).

With the colored part 25 thus provided, in the case of the extension condition the colored part 25 is exposed and, hence, the colored part 25 can be visually confirmed. This makes it possible to securely recognize that the puncture instrument 2A is a used one. Therefore, reuse of a used puncture instrument 2A by mistake can be prevented more assuredly.

While the puncture instrument according to the present invention has been described referring to the embodiments shown in the drawings, the invention is not limited to the embodiments. The components of the puncture instrument can be replaced by those of arbitrary configurations which can exhibit functions the same as or equivalent to the above-mentioned. Besides, arbitrary structures may be added to the constitution of the present invention.

In addition, the puncture instrument according to the present invention may be a combination of arbitrary two or more configurations (features) of the above-described embodiments.

For example, while the projected portion and the recessed portion functioning as the extension condition maintaining means include the projected portion arranged as part of the extension part and the recessed portion arranged as part of the casing in the first embodiment described above, a configuration may be adopted in which the projected portion is arranged as part of the casing and the recessed portion is arranged as part of the extension part, as in the second embodiment.

Besides, while the projected portion and the recessed portion functioning as the extension condition maintaining means include the projected portion arranged as part of the casing and the recessed portion arranged as part of the extension part in the second embodiment described above, a configuration may be adopted in which the projected portion may be arranged as part of the extension part and the recessed portion is arranged as part of the casing, as in the first embodiment.

In addition, "normal attachment" means such an attachment of the puncture instrument to the puncture device that predetermined functions of the puncture device can be exhibited after the attachment. More specifically, the "normal attachment" means that the puncture instrument is mounted in a predetermined position so that the puncturing operation of the puncture device is performed normally, through fixation of the needle hub of the puncture instrument to the distal end of the plunger of the puncture device.

### Industrial Applicability

The puncture instrument according to the present invention is a puncture instrument to be used in the state of being detachably attached to an attaching part of a puncture device, including: a puncture needle which has a needle member provided with a sharp needle tip at its distal end, and a needle hub fixed to a proximal portion of the needle member; a casing adapted to store the puncture needle movably in the longitudinal direction thereof; and an extension part movably disposed on the casing and moved relative to the casing so as to allow the length of the casing on the proximal side to extend; wherein in a non-extension condition where the extension part is not extended, normal attachment to the puncture device is possible, and in an extension condition where the extension part is extended, normal attachment to the puncture device is impossible. Therefore, an unused puncture instrument is in the non-extension condition, while a used puncture instrument is in the extension condition. In the case where it is attempted to attach the puncture instrument in the used state (in the extension condition) to the puncture device, the length of the puncture instrument on the proximal side has been enlarged by an amount corresponding to the increase in the length of the extension part and, therefore, normal attachment of the puncture instrument to the puncture device is impossible. Thus, when the puncture instrument is a used one, the puncture instrument is securely prevented from being again attached to the puncture device; therefore, reuse of the used puncture instrument is also prevented assuredly. In addition, of the puncture instruments, an unused one and a used one are different from each other in the degree of extension of the extension part relative to the casing and, hence, in overall length. This ensures that the state of each puncture instrument, specifically, whether the puncture instrument is an unused one or a used one, can be securely discriminated on a visual basis. Therefore, reuse of a used puncture instrument by mistake can be prevented from occurring. Accordingly, the puncture instrument of the present invention has industrial applicability.

## Claims

1. A puncture instrument to be used in the state of being detachably attached to an attaching part of a puncture device, comprising:
a puncture needle which has a needle member provided with a sharp needle tip at its distal end, and a needle hub fixed to a proximal portion of the needle member;
a casing adapted to store the puncture needle movably in the longitudinal direction thereof; and
an extension part movably disposed on the casing and moved relative to the casing so as to allow the length of the casing on the proximal side to extend;
wherein in a non-extension condition where the extension part is not extended, normal attachment to the puncture device is possible, and in an extension condition where the extension part is extended, normal attachment to the puncture device is impossible.

2. The puncture instrument according to claim 1,
wherein the puncture device has a plunger adapted to be connected to the needle hub when the puncture instrument is attached to the attaching part; and
in the extension condition, the increase in the length of the extension part prevents the needle hub from reaching the plunger, and the needle hub and the plunger cannot be connected to each other.

3. The puncture instrument according to claim 1, wherein the extension part is brought into the extension condition when the puncture instrument, once attached to the attaching part in the non-extension condition, is detached from the attaching part.

4. The puncture instrument according to claim 1, comprising non-extension condition maintaining means for maintaining the non-extension condition.

5. The puncture instrument according to claim 4, wherein the non-extension condition maintaining means includes a projected portion provided in one of the casing and the extension part, and a recessed portion which is provided in the other of the casing and the extension part and with which the projected portion is adapted to be engaged.

6. The puncture instrument according to claim 1, comprising extension condition maintaining means for maintaining the extension condition.

7. The puncture instrument according to claim 6, wherein the extension condition maintaining means includes a projected portion provided in one of the casing and the extension part, and a recessed portion which is provided in the other of the casing and the extension part and with which the projected portion is adapted to be engaged.

8. The puncture instrument according to claim 1,
wherein the extension part has a first engaging portion adapted to be engaged with the attaching part when the puncture instrument is attached to the attaching part, and a second engaging portion adapted to be engaged with the casing in the non-extension condition; and
an engaging force between the first engaging portion and the attaching part is greater than an engaging force between the second engaging portion and the casing.

9. The puncture instrument according to claim 1, wherein the extension part and the casing are tubular in shape.

10. The puncture instrument according to claim 9, wherein the extension part is disposed so as to be slidable on an inner peripheral part of the casing.

11. The puncture instrument according to claim 9, wherein the extension part is disposed so as to be slidable on an outer peripheral part of the casing.

12. The puncture instrument according to claim 1, wherein the casing and the extension part have different colors.

13. The puncture instrument according to claim 1, wherein the casing has a colored part having a portion which is covered with the extension part in the non-extension condition and is exposed in the extension condition, the portion being colored.
